# EUROPEAN PATENT APPLICATION

(11) **EP 4 218 781 A1**
(43) Date of publication of application: **02.08.2023**
(21) Application number: 23174648.8
(22) Date of filing: 14.06.2021
(51) Int. Cl.: A61K 35/744, A61P 3/00, A61P 21/00

(54) **COMPOSITION COMPRISING LACTOBACILLUS BACTERIUM-DERIVED VESICLE FOR PREVENTING OR TREATING METABOLIC DISEASE OR MUSCULAR DISEASE**

(30) Priority: 16.06.2020 KR 20200072685; 08.06.2021 KR 20210073948
(62) Divisional of application: 21805830.3
(71) Applicant: MD Healthcare Inc., Seoul 03923 (KR)
(72) Inventor: KIM, Yoon-Keun, 10908 Paju-si, Gyeonggi-do (KR)
(74) Representative: Dehns

(57) **Abstract**

The present invention relates to a composition for preventing or treating a metabolic disease or muscle disease, which comprises vesicles derived from bacteria of the genus *Lactobacillus.* The inventors confirmed that, when vesicles derived from bacteria of the genus *Lactobacillus* were administered into an animal model with a metabolic disease caused by a high fat, the vesicles effectively inhibit metabolic diseases caused by a high fat diet, and increase homeostasis to metabolic stress by activating AMPK signaling in myocytes, and therefore, the vesicles derived from bacteria of the genus *Lactobacillus* according to the present invention can be effectively used in development of pharmaceuticals or health functional foods for preventing, improving symptoms or treating metabolic diseases such as obesity and muscle diseases.

## Description

### [Technical Field]

The present invention relates to a composition for preventing, improving, or treating a metabolic disease or muscle disease, comprising vesicles derived from bacteria of the genus *Lactobacillus* as an active ingredient, and the like.

This application claims priority to and the benefit of Korean Patent Application Nos. 10-2020-0072685 and 10-2021-0073948 filed in the Korean Intellectual Property Office on June 16, 2020 and June 8, 2021, respectively, and all the contents disclosed in the specification and drawings of the applications are incorporated in this application.

### [Background Art]

Metabolic diseases are disease occurring due to metabolic disorders in the body. Generally, metabolic diseases are caused by an imbalance of carbohydrates, lipids, proteins, vitamins, electrolytes and water, and include, for example, obesity, diabetes, hyperlipidemia, arteriosclerosis, fatty liver and high blood pressure. Among them, type 2 diabetes relating to obesity is a type of diabetes mainly occurring in adulthood and is characterized by resistance to insulin. When the insulin receptor itself is reduced or its sensitivity is decreased, or there is a problem with the secondary messenger causing glycogen synthesis in cells, sensitivity to insulin may decrease. Accordingly, type 2 diabetes is called non-insulin-dependent diabetes and accounts for 85 to 90% of diabetes.

Bacteria of the genus *Lactobacillus* is known as representative good bacteria secreting lactic acid. Among the *Lactobacillus* species, *Lactobacillus paracasei* is a gram-positive species of the class Bacilli, which grows well in an anaerobic environment as well as an aerobic environment and is known as a symbiotic bacterium in the vagina of women. Bacteria secrete extracellular vesicles (EVs) often called nanovesicles, as proteolipids with a double membrane structure, into an extracellular environment for intracellular exchange of proteins, lipids or genes. Vesicles derived from gram-positive bacteria such as *Lactobacillus paracasei* include peptidoglycan and lipoteichoic acid, which are constituents of bacterial cell walls, in addition to bacteria-derived proteins and nucleic acids.

However, vesicles secreted from bacteria of the genus *Lactobacillus* or *Lactobacillus paracasei* have not been used to prevent or treat metabolic diseases such as obesity, metabolic syndrome, hyperinsulinemia, hyperlipidemia, hypertriglyceridemia, hypercholesterolemia, dyslipidemia, diabetes, diabetic nephropathy, fatty liver, nonalcoholic steatohepatitis and arteriosclerosis.

### [Disclosure]

### [Technical Problem]

As a result of earnestly conducting a study to evaluate whether vesicles derived from bacteria of the genus *Lactobacillus* such as *Lactobacillus paracasei* and *Lactobacillus rhamnosus* have a therapeutic effect on metabolic diseases, the inventors observed that administration of bacteria of the genus *Lactobacillus-derived* vesicles into animal models with obesity and diabetes caused by a high-fat diet effectively inhibits obesity and diabetes, and based on this, the present invention was completed.

Thus, the present invention is directed to providing a composition for preventing, alleviating, or treating a metabolic disease, comprising vesicles derived from bacteria of the genus *Lactobacillus* as an active ingredient.

In addition, the present invention is directed to providing a composition for preventing, alleviating, or treating a muscle disease, comprising vesicles derived from bacteria of the genus *Lactobacillus* as an active ingredient.

In addition, the present invention is directed to providing a food composition for improving a muscle function, comprising vesicles derived from bacteria of the genus *Lactobacillus* as an active ingredient.

In addition, the present invention is directed to providing a food composition for enhancing exercise performance capacity, comprising vesicles derived from bacteria of the genus *Lactobacillus* as an active ingredient.

However, a technical problem to be achieved by the present invention is not limited to the aforementioned problems, and the other problems that are not mentioned may be clearly understood by a person skilled in the art from the following description.

### [Technical Solution]

To achieve the object of the present invention as described above, the present invention provides a pharmaceutical composition for preventing or treating a metabolic disease, comprising vesicles derived from bacteria of the genus *Lactobacillus* as an active ingredient.

In addition, the present invention provides a food composition for preventing or alleviating a metabolic disease, comprising vesicles derived from bacteria of the genus *Lactobacillus* as an active ingredient.

As an exemplary embodiment of the present invention, the bacteria of the genus *Lactobacillus* may be *Lactobacillus paracasei* or *Lactobacillus rhamnosus,* but is not limited thereto.

As another exemplary embodiment of the present invention, the food composition may be a health functional food composition, but is not limited thereto.

As another exemplary embodiment of the present invention, the metabolic disease may be one or more diseases selected from the group consisting of obesity, metabolic syndrome, hyperinsulinemia, hyperlipidemia, hypertriglyceridemia, hypercholesterolemia, dyslipidemia, diabetes, diabetic nephropathy, diabetic retinopathy, fatty liver, non-alcoholic steatohepatitis and arteriosclerosis.

As another exemplary embodiment of the present invention, the vesicles may have an average diameter of 10 to 1000 nm.

As another exemplary embodiment of the present invention, the vesicles may be isolated from culture solution of bacteria of the genus *Lactobacillus.*

As another exemplary embodiment of the present invention, the vesicles may be obtained using vesicles isolated from a food prepared by adding bacteria of the genus *Lactobacillus.*

As another exemplary embodiment of the present invention, the vesicles may be naturally or artificially secreted from bacteria of the genus *Lactobacillus.*

In addition, the present invention provides a method for preventing or treating a metabolic disease, the method comprising administering the composition to an individual.

In addition, the present invention provides a use of a composition comprising vesicles derived from bacteria of the genus *Lactobacillus* as an active ingredient for preventing or treating metabolic diseases.

In addition, the present invention provides a use of vesicles derived from bacteria of the genus *Lactobacillus* for preparing a drug for preventing or treating a metabolic disease.

In addition, the present invention provides a pharmaceutical composition for preventing or treating a muscle disease, comprising vesicles derived from bacteria of the genus *Lactobacillus* as an active ingredient.

In addition, the present invention provides a food composition for improving a muscle function, comprising vesicles derived from bacteria of the genus *Lactobacillus* as an active ingredient.

In addition, the present invention provides a food composition for enhancing exercise performance capacity, comprising vesicles derived from bacteria of the genus *Lactobacillus* as an active ingredient.

As an exemplary embodiment of the present invention, the bacteria of the genus *Lactobacillus* may be *Lactobacillus paracasei* or *Lactobacillus rhamnosus.*

As another exemplary embodiment of the present invention, the muscle disease may be one or more selected from the group consisting of atony, muscular atrophy, muscular dystrophy, myasthenia, cachexia and sarcopenia.

In addition, the present invention provides a method for preventing or treating a muscle disease, the method comprising administering the composition to an individual.

In addition, the present invention provides a use of a composition comprising vesicles derived from bacteria of the genus *Lactobacillus* as an active ingredient for preventing or treating a muscle disease.

In addition, the present invention provides a use of vesicles derived from bacteria of the genus *Lactobacillus* for preparing a drug for preventing or treating a muscle disease.

In addition, the present invention provides a method for improving a muscle function or enhancing exercise performance capacity, the method comprising administering a composition comprising vesicles derived from bacteria of the genus *Lactobacillus* as an active ingredient to an individual.

In addition, the present invention provides a use of a composition comprising vesicles derived from bacteria of the genus *Lactobacillus* as an active ingredient for improving a muscle function or enhancing exercise performance capacity.

In addition, the present invention provides a use of vesicles derived from bacteria of the genus *Lactobacillus* for preparing a drug for improving a muscle function or enhancing exercise performance capacity.

### [Advantageous Effects]

The inventors confirmed that, when vesicles derived from bacteria of the genus *Lactobacillus* were administered into an animal model with a metabolic disease caused by a high fat western diet, the vesicles not only reduce a body weight and a meal amount, but also effectively inhibit metabolic disorders caused by a western diet, and increase homeostasis to metabolic stress by activating AMPK signaling in myocytes, and the vesicles derived from bacteria of the genus *Lactobacillus* according to the present invention can be effectively used in development of pharmaceuticals or health functional foods for preventing, improving symptoms or treating metabolic diseases and muscle diseases such as obesity, metabolic syndrome, hyperinsulinemia, hyperlipidemia, hypertriglyceridemia, hypercholesterolemia, dyslipidemia, diabetes, diabetic nephropathy, diabetic retinopathy, fatty liver, non-alcoholic steatohepatitis and arteriosclerosis.

### [Description of Drawings]

FIG. 1 is a diagram for analyzing a protein pattern contained in vesicles isolated from *a Lactobacillus paracasei* culture through SDS-PAGE and a silver staining method.
FIG. 2 is a diagram of evaluating the distribution pattern of vesicles after *Lactobacillus paracasei* vesicles are labeled with fluorescence and orally administered.
FIG. 3 is an experimental protocol for confirming an effect of treating a metabolic disease after *Lactobacillus paracasei*-derived vesicles are orally administered into a mouse model with a metabolic disease induced by a high fat diet.
FIG. 4 is a result of observing a body weight change after *Lactobacillus paracasei-*derived vesicles are directly administered orally into a mouse model with a metabolic disease induced by a high fat diet.
FIG. 5 is an experimental protocol for evaluating concentration dependency for an effect of treating a metabolic disease after various concentrations of *Lactobacillus paracasei-*derived vesicles (MDH-001) are orally administered into a mouse model with a metabolic disease induced by a high fat diet.
FIG. 6 is a result of evaluating a body weight change and food intake after various concentrations *of Lactobacillus paracasei*-derived vesicles are directly administered orally into a mouse model with a metabolic disease induced by a high fat diet.
FIG. 7 is a result of evaluating the degree of expression of a GLUT4 transporter, which is a glucose receptor, after metformin, E. coli-derived vesicles (EcEVs) or *Lactobacillus paracasei*-derived vesicles (L-EVs) are administered into myocytes.
FIG. 8 is a result of evaluating the degree of glucose uptake in myocytes after insulin, metformin, E. coli-derived vesicles (EcEVs) or *Lactobacillus paracasei*-derived vesicles (L-EVs) are administered into myocytes.
FIG. 9 is a result of evaluating AMPK activation for 1 hour after myocytes are treated with insulin, metformin, various concentrations *of Lactobacillus paracasei*-derived vesicles *(L. paracasei* EVs).
FIG. 10 is a result of evaluating AMPK activation for 1 hour after myocytes are treated with insulin, metformin, various concentrations of *Lactobacillus rhamnosus*-derived vesicles (*L. rhamnosus* EVs).

### [Modes of the Invention]

The inventors confirmed that, in a mouse model with a metabolic disease induced by a high fat diet, when vesicles derived from bacteria of the genus *Lactobacillus* were administered orally, food intake decreased, and a body weight increase was inhibited in a dose-dependent manner.

In still another embodiment of the present invention, in a mouse model with a metabolic disease induced by a high fat diet, it was confirmed that when vesicles derived from bacteria of the genus *Lactobacillus* were administered orally, a fasting blood sugar level is suppressed.

In yet another embodiment of the present invention, when vesicles derived from bacteria of the genus *Lactobacillus* were administered into myocytes, it was confirmed that the expression of GLUT4, which is an indicator of insulin resistance, and glucose uptake in myocytes are increased by vesicles.

In yet another embodiment of the present invention, it was confirmed that, when vesicles derived from bacteria of the genus *Lactobacillus* were administered into myocytes, a mechanism of increasing insulin resistance by vesicles is associated with AMPK activation.

Thus, the present invention provides a pharmaceutical composition for preventing, alleviating, or treating a metabolic disease or a muscle disease, comprising vesicles derived from bacteria of the genus *Lactobacillus* as an active ingredient.

In the present invention, the bacteria of the genus *Lactobacillus* may be *Lactobacillus paracasei* or *Lactobacillus rhamnosus,* but is not limited thereto.

As used herein, the term "vesicle" refers to a structure formed of a nano-sized membrane secreted from various bacteria, and in the present invention, the term collectively refers to all structures formed of a membrane naturally secreted from bacteria of the genus *Lactobacillus,* or artificially produced. The vesicles may be isolated from a culture solution including bacterial cells of bacteria of the genus *Lactobacillus* by using one or more methods selected from the group consisting of centrifugation, ultra-high speed centrifugation, high pressure treatment, extrusion, sonication, cell lysis, homogenization, freezing-thawing, electroporation, mechanical decomposition, chemical treatment, filtration by filter, gel filtration chromatography, free-flow electrophoresis, and capillary electrophoresis. Further, a process such as washing for removing impurities and concentration of obtained vesicles may be further included.

The vesicles of the present invention may be isolated from a culture solution of bacteria of the genus *Lactobacillus* or a food prepared by adding bacteria of the genus *Lactobacillus,* and the vesicles may be naturally or artificially secreted from bacteria of the genus *Lactobacillus,* but are not limited thereto.

The method for isolating vesicles from the culture solution or fermented food of the bacteria of the genus *Lactobacillus* of the present invention is not particularly limited as long as the vesicles are included. For example, vesicles may be isolated using a method such as centrifugation, ultra-high speed centrifugation, filtration by a filter, gel filtration chromatography, free-flow electrophoresis, or capillary electrophoresis, and a combination thereof, and further, a process such as washing to remove impurities and concentration of obtained vesicles may be further included.

In the present invention, vesicles isolated by the method may have an average diameter 10 to 1000 nm, 10 to 900 nm, 10 to 800 nm, 10 to 700 nm, 10 to 600 nm, 10 to 500 nm, 10 to 400 nm, 10 to 300 nm, 10 to 200 nm, 20 to 1000 nm, 20 to 900 nm, 20 to 800 nm, 20 to 700 nm, 20 to 600 nm, 20 to 500 nm, 20 to 400 nm, 20 to 300 nm, 20 to 200 nm, 30 to 1000 nm, 30 to 900 nm, 30 to 800 nm, 30 to 700 nm, 30 to 600 nm, 30 to 500 nm, 30 to 400 nm, 30 to 300 nm, 30 to 200 nm, 40 to 1000 nm, 40 to 900 nm, 40 to 800 nm, 40 to 700 nm, 40 to 600 nm, 40 to 500 nm, 40 to 400 nm, 40 to 300 nm, 40 to 200 nm, 20 to 180 nm, 30 to 180 nm, 40 to 180 nm, 20 to 170 nm, 20 to 160 nm, 40 to 160 nm, 45 to 155 nm, or 50 to 150 nm, but the average diameter is not limited thereto.

The term "metabolic disease" used herein encompasses diseases occurring due to metabolic disorders in the body. Generally, metabolic diseases are caused by an imbalance of carbohydrates, lipids, proteins, vitamins, electrolytes and water, and include, for example, obesity, metabolic syndrome, hyperinsulinemia, hyperlipidemia, hypertriglyceridemia, hypercholesterolemia, dyslipidemia, diabetes, diabetic nephropathy, diabetic retinopathy, fatty liver, non-alcoholic steatohepatitis and arteriosclerosis.

The term "muscle disease" used herein may refer to a disease caused by muscle wasting or degeneration, but the present invention is not limited thereto. In the present invention, the muscle disease may be one or more selected from the group consisting of atony, muscular atrophy, muscular dystrophy, myasthenia, cachexia and sarcopenia, but the present invention is not limited thereto. Muscle wasting and degeneration are caused by genetic factors, acquired factors, aging, etc. The composition of the present invention has an effect of promoting a muscle increase, and the muscle is not limited in type. A muscle increase is an improvement in performance of a muscle, particularly, among body components, and muscle mass may be increased by physical exercise and improved endurance by a method of administering a material having a muscle increasing effect into the body.

The amount of the vesicles in the composition of the present invention may be appropriately adjusted depending on the symptoms of a disease, the degree of progression of symptoms, the condition of a patient, and the like, and may range from, for example, 0.0001 wt% to 99.9 wt% or 0.001 wt% to 50 wt% with respect to a total weight of the composition, but the present invention is not limited thereto. The amount ratio is a value based on the amount of dried product from which a solvent is removed.

The pharmaceutical composition according to the present invention may further include a suitable carrier, excipient, and diluent which are commonly used in the preparation of pharmaceutical compositions. The excipient may be, for example, one or more selected from the group consisting of a diluent, a binder, a disintegrant, a lubricant, an adsorbent, a humectant, a film-coating material, and a controlled release additive.

The pharmaceutical composition according to the present invention may be used by being formulated, according to commonly used methods, into a form such as powders, granules, sustained-release-type granules, enteric granules, liquids, eye drops, elixirs, emulsions, suspensions, spirits, troches, aromatic water, lemonades, tablets, sustained-release-type tablets, enteric tablets, sublingual tablets, hard capsules, soft capsules, sustained-release-type capsules, enteric capsules, pills, tinctures, soft extracts, dry extracts, fluid extracts, injections, capsules, perfusates, or a preparation for external use, such as plasters, lotions, pastes, sprays, inhalants, patches, sterile injectable solutions, or aerosols. The preparation for external use may have a formulation such as creams, gels, patches, sprays, ointments, plasters, lotions, liniments, pastes, or cataplasmas.

As the carrier, the excipient, and the diluent that may be included in the pharmaceutical composition according to the present invention, lactose, dextrose, sucrose, oligosaccharides, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil may be used.

For formulation, commonly used diluents or excipients such as fillers, thickeners, binders, wetting agents, disintegrants, and surfactants are used.

As additives of tablets, powders, granules, capsules, pills, and troches according to the present invention, excipients such as corn starch, potato starch, wheat starch, lactose, white sugar, glucose, fructose, D-mannitol, precipitated calcium carbonate, synthetic aluminum silicate, dibasic calcium phosphate, calcium sulfate, sodium chloride, sodium hydrogen carbonate, purified lanolin, microcrystalline cellulose, dextrin, sodium alginate, methyl cellulose, sodium carboxymethylcellulose, kaolin, urea, colloidal silica gel, hydroxypropyl starch, hydroxypropyl methylcellulose (HPMC) 1928, HPMC 2208, HPMC 2906, HPMC 2910, propylene glycol, casein, calcium lactate, and *Primojel^{®};* and binders such as gelatin, Arabic gum, ethanol, agar powder, cellulose acetate phthalate, carboxymethylcellulose, calcium carboxymethylcellulose, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, purified shellac, starch, hydroxypropyl cellulose, hydroxypropyl methylcellulose, polyvinyl alcohol, and polyvinylpyrrolidone may be used, and disintegrants such as hydroxypropyl methylcellulose, corn starch, agar powder, methylcellulose, bentonite, hydroxypropyl starch, sodium carboxymethylcellulose, sodium alginate, calcium carboxymethylcellulose, calcium citrate, sodium lauryl sulfate, silicic anhydride, 1-hydroxypropylcellulose, dextran, ionexchange resin, polyvinyl acetate, formaldehyde-treated casein and gelatin, alginic acid, amylose, guar gum, sodium bicarbonate, polyvinylpyrrolidone, calcium phosphate, gelled starch, Arabic gum, amylopectin, pectin, sodium polyphosphate, ethyl cellulose, white sugar, magnesium aluminum silicate, a di-sorbitol solution, and light anhydrous silicic acid; and lubricants such as calcium stearate, magnesium stearate, stearic acid, hydrogenated vegetable oil, talc, lycopodium powder, kaolin, Vaseline, sodium stearate, cacao butter, sodium salicylate, magnesium salicylate, polyethylene glycol (PEG) 4000, PEG 6000, liquid paraffin, hydrogenated soybean oil (Lubri wax), aluminum stearate, zinc stearate, sodium lauryl sulfate, magnesium oxide, Macrogol, synthetic aluminum silicate, silicic anhydride, higher fatty acids, higher alcohols, silicone oil, paraffin oil, polyethylene glycol fatty acid ether, starch, sodium chloride, sodium acetate, sodium oleate, dl-leucine, and light anhydrous silicic acid may be used.

As additives of liquids according to the present invention, water, dilute hydrochloric acid, dilute sulfuric acid, sodium citrate, monostearic acid sucrose, polyoxyethylene sorbitol fatty acid esters (twin esters), polyoxyethylene monoalkyl ethers, lanolin ethers, lanolin esters, acetic acid, hydrochloric acid, ammonia water, ammonium carbonate, potassium hydroxide, sodium hydroxide, prolamine, polyvinylpyrrolidone, ethylcellulose, and sodium carboxymethylcellulose may be used.

In syrups according to the present invention, a white sugar solution, other sugars or sweeteners, and the like may be used, and as necessary, a fragrance, a colorant, a preservative, a stabilizer, a suspending agent, an emulsifier, a viscous agent, or the like may be used.

In emulsions according to the present invention, purified water may be used, and as necessary, an emulsifier, a preservative, a stabilizer, a fragrance, or the like may be used.

In suspensions according to the present invention, suspending agents such as acacia, tragacanth, methylcellulose, carboxymethylcellulose, sodium carboxymethylcellulose, microcrystalline cellulose, sodium alginate, hydroxypropyl methylcellulose (HPMC) 1828, HPMC 2906, HPMC 2910, and the like may be used, and as necessary, a surfactant, a preservative, a stabilizer, a colorant, and a fragrance may be used.

Injections according to the present invention may include: solvents such as distilled water for injection, a 0.9% sodium chloride solution, Ringer's solution, a dextrose solution, a dextrose+sodium chloride solution, PEG, lactated Ringer's solution, ethanol, propylene glycol, non-volatile oil-sesame oil, cottonseed oil, peanut oil, soybean oil, corn oil, ethyl oleate, isopropyl myristate, and benzene benzoate; cosolvents such as sodium benzoate, sodium salicylate, sodium acetate, urea, urethane, monoethylacetamide, butazolidine, propylene glycol, the Tween series, amide nicotinate, hexamine, and dimethylacetamide; buffers such as weak acids and salts thereof (acetic acid and sodium acetate), weak bases and salts thereof (ammonia and ammonium acetate), organic compounds, proteins, albumin, peptone, and gums; isotonic agents such as sodium chloride; stabilizers such as sodium bisulfite (NaHSO₃) carbon dioxide gas, sodium metabisulfite (Na₂S₂O₅), sodium sulfite (Na₂SO₃), nitrogen gas (N₂), and ethylenediamine tetraacetic acid; sulfating agents such as 0.1% sodium bisulfide, sodium formaldehyde sulfoxylate, thiourea, disodium ethylenediaminetetraacetate, and acetone sodium bisulfite; a pain relief agent such as benzyl alcohol, chlorobutanol, procaine hydrochloride, glucose, and calcium gluconate; and suspending agents such as sodium CMC, sodium alginate, Tween 80, and aluminum monostearate.

In suppositories according to the present invention, bases such as cacao butter, lanolin, Witepsol, polyethylene glycol, glycerogelatin, methylcellulose, carboxymethylcellulose, a mixture of stearic acid and oleic acid, Subanal, cottonseed oil, peanut oil, palm oil, cacao butter + cholesterol, lecithin, lanette wax, glycerol monostearate, Tween or span, imhausen, monolan(propylene glycol monostearate), glycerin, Adeps solidus, buytyrum Tego-G, cebes Pharma 16, hexalide base 95, cotomar, Hydrokote SP, S-70-XXA, S-70-XX75(S-70-XX95), Hydrokote 25, Hydrokote 711, idropostal, massa estrarium (A, AS, B, C, D, E, I, T), masa-MF, masupol, masupol-15, neosuppostal-N, paramount-B, supposiro OSI, OSIX, A, B, C, D, H, L, suppository base IV types AB, B, A, BC, BBG, E, BGF, C, D, 299, suppostal N, Es, Wecoby W, R, S, M, Fs, and tegester triglyceride matter (TG-95, MA, 57) may be used.

Solid preparations for oral administration include tablets, pills, powders, granules, capsules, and the like, and such solid preparations are formulated by mixing the composition with at least one excipient, e.g., starch, calcium carbonate, sucrose, lactose, gelatin, and the like. In addition to simple excipients, lubricants such as magnesium stearate and talc are also used.

Examples of liquid preparations for oral administration include suspensions, liquids for internal use, emulsions, syrups, and the like, and these liquid preparations may include, in addition to simple commonly used diluents, such as water and liquid paraffin, various types of excipients, for example, a wetting agent, a sweetener, a fragrance, a preservative, and the like. Preparations for parenteral administration include an aqueous sterile solution, a non-aqueous solvent, a suspension, an emulsion, a freeze-dried preparation, and a suppository. Nonlimiting examples of the non-aqueous solvent and the suspension include propylene glycol, polyethylene glycol, a vegetable oil such as olive oil, and an injectable ester such as ethyl oleate.

The pharmaceutical composition according to the present invention is administered in a pharmaceutically effective amount. In the present invention, "the pharmaceutically effective amount" refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration route, excretion rate, treatment period, and simultaneously used drugs, and factors well known in other medical fields.

The composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this may be easily determined by those of ordinary skill in the art.

The pharmaceutical composition of the present invention may be administered to an individual via various routes. All administration methods can be predicted, and the pharmaceutical composition may be administered via, for example, oral administration, subcutaneous injection, intravenous injection, intramuscular injection, intrathecal (space around the spinal cord) injection, sublingual administration, administration via the buccal mucosa, intrarectal insertion, intravaginal insertion, ocular administration, intra-aural administration, intranasal administration, inhalation, spraying via the mouth or nose, transdermal administration, percutaneous administration, or the like.

The pharmaceutical composition of the present invention is determined depending on the type of a drug, which is an active ingredient, along with various related factors such as a disease to be treated, administration route, the age, gender, and body weight of a patient, and the severity of diseases. Specifically, the effective amount of the composition according to the present invention may vary depending on the patient's age, sex, and body weight, and generally, 0.001 to 150 mg of the composition and preferably, 0.01 to 100 mg of the composition, per 1 kg of the body weight, may be administered daily or every other day or may be administered once to three times a day. However, since the effective amount may be increased or decreased depending on the administration route, the severity of obesity, gender, body weight, age, and the like, the dosage is not intended to limit the scope of the present invention in any way.

As used herein, the "subject" refers to a subject in need of treatment of a disease, and more specifically, refers to a mammal such as a human or a non-human primate, a mouse, a rat, a dog, a cat, a horse, and a cow, but the present invention is not limited thereto.

As used herein, the "administration" refers to providing a subject with a predetermined composition of the present invention by using an arbitrary appropriate method.

The term "prevention" as used herein means all actions that inhibit or delay the onset of a target disease. The term "treatment" as used herein means all actions that alleviate or beneficially change a target disease and abnormal metabolic symptoms caused thereby via administration of the pharmaceutical composition according to the present invention. The term "improvement" as used herein means all actions that reduce the degree of parameters related to a target disease, e.g., symptoms via administration of the composition according to the present invention.

In addition, the present invention provides a food composition for preventing or alleviating a metabolic disease or a muscle disease, comprising vesicles derived from bacteria of the genus *Lactobacillus* as an active ingredient.

In addition, the present invention provides a food composition for improving a muscle function, comprising vesicles derived from bacteria of the genus *Lactobacillus* as an active ingredient.

In addition, the present invention provides a food composition for enhancing exercise performance capacity, comprising vesicles derived from bacteria of the genus *Lactobacillus* as an active ingredient.

The improvement in muscle function refers to strengthening of the intrinsic properties or functions of muscles such as increasing muscle mass, improving muscular strength and recovering muscle fatigue.

The "muscle" used herein encompasses a sinew, muscle and a tendon, and the "muscle function" refers to an ability to exert a force by muscle contraction and includes muscular strength which is an ability to exert maximum contracting power to overcome resistance, muscular endurance which is an ability to exhibit how long or how many times muscle can repeat contraction and relaxation at a given weight, and explosive muscle strength which is an ability to exert a strong force within a short period of time. Such muscle functions are controlled by the liver, and proportional to muscle mass. The term "improving a muscle function" refers to enhancing a muscle function for the better.

The term "enhancing exercise performance capacity" means an effect of enhancing a muscle function such as muscular strength or muscular endurance, and preferably, an increase in muscular strength or muscular endurance. Accordingly, the composition has an effect of increasing exercise endurance, strengthening muscular strength, enhancing sense of balance, and/or enhancing exercise adaptation. One of the representative methods for improving exercise performance capacity by promoting energy consumption is a method of generating ATP energy by increasing fatty acid oxidation caused by mitochondria. AMPK, pAMPK, PPAR6, ERRα, Tfam, and the like are known as factors related to the improvement in exercise performance capacity.

The food composition may be a health functional food composition, but is not limited thereto.

The vesicles according to the present invention may be used by adding an active ingredient as is to food or may be used together with other foods or food ingredients, but may be appropriately used according to a typical method. The mixed amount of the active ingredient may be suitably determined depending on the purpose of use thereof (for prevention or alleviation). In general, when a food or beverage is prepared, the composition of the present invention is added in an amount of 15 wt% or less, preferably 10 wt% or less based on the raw materials. However, for long-term intake for the purpose of health and hygiene or for the purpose of health control, the amount may be less than the above-mentioned range, and the vesicles have no problem in terms of stability, so the active ingredient may be used in an amount more than the above-mentioned range.

The type of food is not particularly limited. Examples of food to which the material may be added include meats, sausage, bread, chocolate, candies, snacks, confectioneries, pizza, instant noodles, other noodles, gums, dairy products including ice creams, various soups, beverages, tea, drinks, alcoholic beverages, vitamin complexes, and the like, and include all health functional foods in a typical sense.

The health beverage composition according to the present invention may contain various flavors or natural carbohydrates, and the like as additional ingredients as in a typical beverage. The above-described natural carbohydrates may be monosaccharides such as glucose and fructose, disaccharides such as maltose and sucrose, polysaccharides such as dextrin and cyclodextrin, and sugar alcohols such as xylitol, sorbitol, and erythritol. As a sweetener, it is possible to use a natural sweetener such as thaumatin and stevia extract, a synthetic sweetener such as saccharin and aspartame, and the like. The proportion of the natural carbohydrates is generally about 0.01 to 0.20 g, or about 0.04 to 0.10 g per 100 ml of the composition of the present invention.

In addition to the aforementioned ingredients, the composition of the present invention may contain various nutrients, vitamins, electrolytes, flavors, colorants, pectic acids and salts thereof, alginic acid and salts thereof, organic acids, protective colloid thickeners, pH adjusters, stabilizers, preservatives, glycerin, alcohols, carbonating agents used in carbonated drinks, and the like. In addition, the composition of the present invention may contain flesh for preparing natural fruit juice, fruit juice drinks, and vegetable drinks. These ingredients may be used either alone or in combinations thereof. The proportion of these additives is not significantly important, but is generally selected within a range of 0.01 to 0.20 part by weight per 100 parts by weight of the composition of the present invention.

Hereinafter, preferred Examples for helping the understanding of the present invention will be suggested. However, the following Examples are provided only to more easily understand the present invention, and the contents of the present invention are not limited by the following Examples.

### [Examples]

### Example 1: Isolation of vesicles derived from Lactobacillus paracasei

To isolate vesicles derived from representative bacteria of the genus *Lactobacillus* such as *Lactobacillus paracasei* and *Lactobacillus rhamnosus*-derived vesicles (extracellular vesicles; EVs), the *Lactobacillus* strains were inoculated into an MRS (de Man-Rogosa and Sharpe) medium, incubated at 37 °C and 200 rpm to have an absorbance (OD₆₀₀ₙₘ) of 1.0 to 1.5, and reinoculated into a Luria Bertani (LB) medium and incubated. Then, a supernatant from which bacterial cells had been removed was obtained by recovering the culture solution including bacterial cells and performing centrifugation at 4°C and 10,000 g for 20 minutes. The obtained supernatant was again filtered using a 0.22 µm filter, and the filtered supernatant was concentrated to a volume of 50 mL or less using a 100 kDa Pellicon 2 Cassette filter membrane (Merck Millipore) and a MasterFlex pump system (Cole-Parmer). A vesicle derived from bacteria of *Lactobacillus* was isolated by filtering the concentrated supernatant again using a 0.22 µm filter. In the following examples, experiments were performed using the isolated vesicle.

### Example 2. Characterization of vesicles derived from Lactobacillus paracasei

To confirm the characteristics of vesicles derived from *Lactobacillus paracasei* (extracellular vesicles; EVs) acquired by the method of Example 1, protein quantification, measurement of particle size and distribution and protein pattern analysis were performed.

As a result of protein quantification using a BCA method, it was confirmed that an average protein concentration is 3 to 5 mg/mL. In addition, to confirm the size and distribution of vesicles derived from *Lactobacillus paracasei,* nanoparticle tracking analysis (NTA) was performed using an NS300 instrument (Malvern Panalytical). After diluting the concentration of a sample to 1 mg/mL based on a protein concentration, the sample was diluted 100 to 2,000 times to adjust a 'particles/frame' value to 20 to 100.

As a result, it was confirmed that the extracellular vesicles have a size of 50 to 150 nm, and contain 1.0E+11 particles/mL or more per 1 mL.

In addition, to confirm a protein pattern of vesicles derived from *Lactobacillus paracasei,* sodium dodecyl sulphate-polyacrylamide gel electrophoresis (SDS-PAGE) and silver staining were performed.

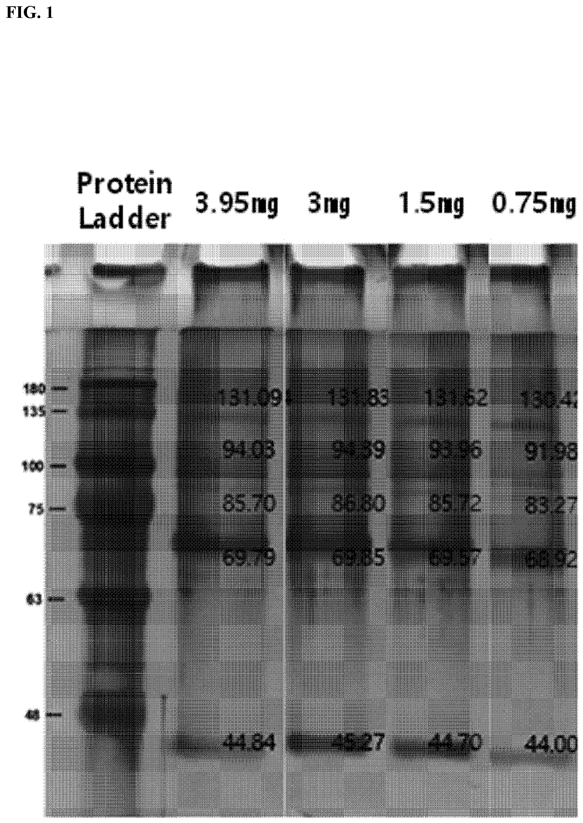

As shown in FIG. 1, it was confirmed that the vesicles derived from *Lactobacillus paracasei* are observed in five main bands (near 130, 95, 85, 70 and 45 kDa).

### Example 3. Pharmacokinetic characteristics of vesicles derived from Lactobacillus paracasei

To confirm the pharmacokinetic properties such as systemic absorption and distribution patterns in oral administration of vesicles derived from *Lactobacillus paracasei,* a distribution in the body was confirmed by the following method.

Vesicles derived from *Lactobacillus paracasei* labeled with the phosphor Cy7 were orally administered into mice, and to confirm the infiltration pattern into various organs after 0, 1, 3, 6, 24, 32, 48, 56 and 72 hours, blood, the heart, lungs, liver, kidneys, spleen, gastrointestinal tract and brain were obtained. A fluorescent signal from the obtained tissue was observed using optical imaging equipment (Davinch-Invivo Fluoro Chemi (western); Davinch-K).

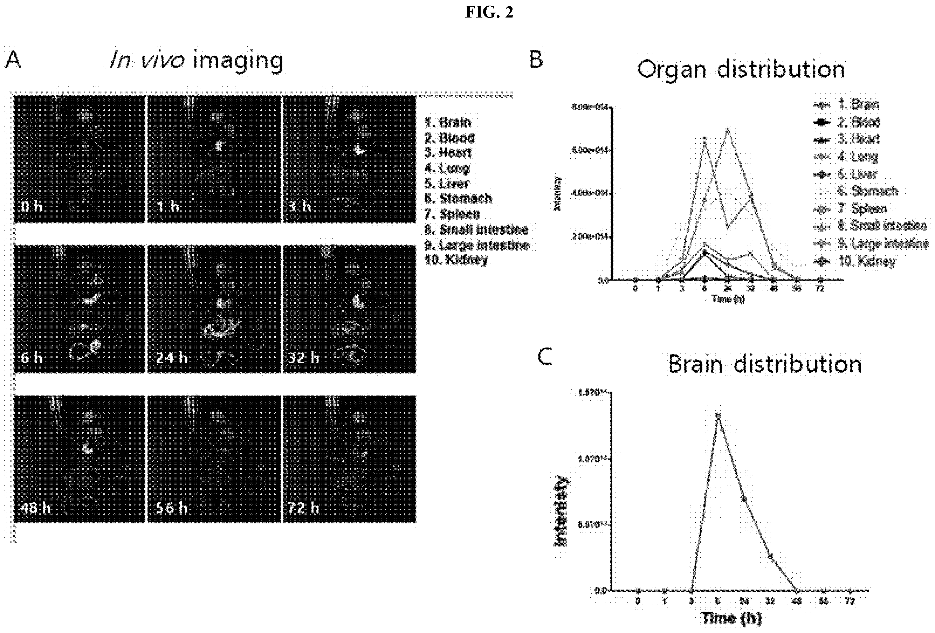

As shown in FIG. 2, it was confirmed that, after the administration of vesicles derived from *Lactobacillus paracasei,* the vesicles were absorbed into the stomach within 1 hour, distributed into the stomach, small and large intestines and lungs at 3 hours, and also distributed in the liver and brain after 3 hours. That is, from the result of continuously observing fluorescent signals of extracellular vesicles in various tissues from 32 to 48 hours, it was confirmed that in the case of oral administration of extracellular vesicles, the vesicles are distributed throughout the entire body including the stomach, intestines, lungs, liver and brain until 48 hours.

### Example 4. Obesity treating effect of vesicles derived from Lactobacillus paracasei in mice with metabolic diseases induced by high fat diet

To confirm whether the vesicles derived from *Lactobacillus paracasei* obtained in Example 1 inhibit a metabolic disease induced by a high fat diet, an experiment was performed by the following method.

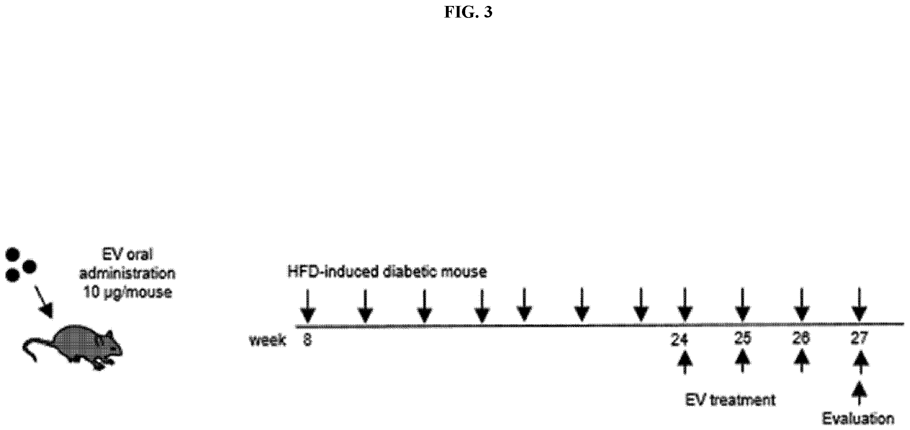

Specifically, according to the experimental protocol shown in FIG. 3, a normal mouse fed a regular diet (RD) and a mouse fed a high fat diet (HFD) for 4 months to induce a metabolic disease were prepared. The vesicles derived from *Lactobacillus paracasei* obtained in Example 1 were administered into the metabolic disease model at 10 µg per mouse every 2 days for 3 weeks. Here, a negative control (NC) was fed a normal diet, and a positive control (obese) was fed a high fat diet. As control drugs, vesicles derived from *Lactobacillus rhamnosus (L. rhamnosus),* vesicles derived from *Lactobacillus lactis (L. lactis),* and vesicles derived from *Proteus mirabilis (P. mirabilis)* were orally administered at the same dose as the vesicles derived from *Lactobacillus paracasei.* In addition, while the vesicles were administered every 2 days, a body weight was measured.

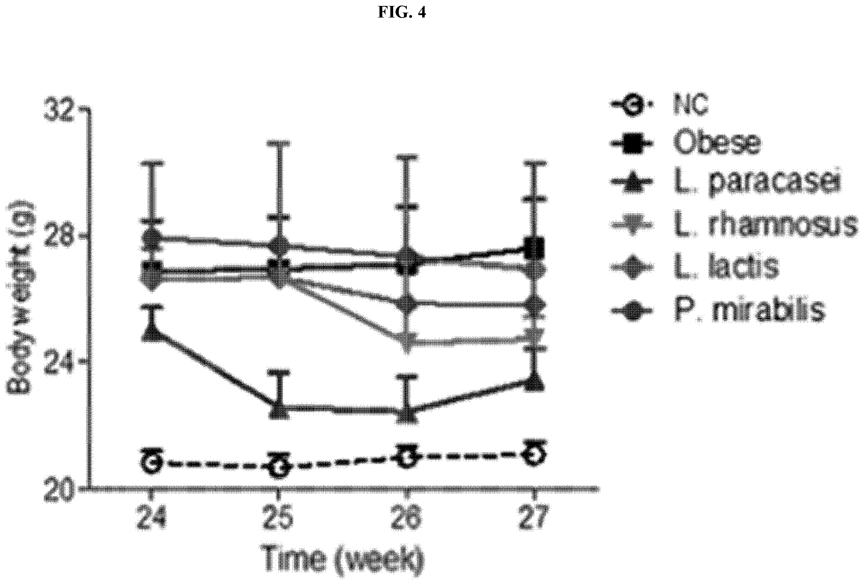

As shown in FIG. 4, compared to a high fat diet-fed positive control (obese) to which a drug was not administered, in a group to which vesicles derived from *Lactobacillus paracasei (L. paracasei)* were administered and a group to which vesicles derived from *Lactobacillus rhamnosus (L. rhamnosus)* were administered, body weights were reduced, and in the group ingesting vesicles derived from *Lactobacillus paracasei,* a body weight loss was more clearly observed.

However, the group to which vesicles derived from *Proteus mirabilis (P. mirabilis)* were administered showed similar body weights to those of the high fat diet-fed positive control, and compared to the group fed only a high fat diet, the *Proteus mirabilis* group even showed increased body weights, and the *L. lactis*-derived vesicle-administered group showed a slight body weight loss.

### Example 5. Dose dependence on therapeutic effect of vesicles derived from Lactobacillus paracasei in mouse with metabolic disease induced by high fat diet

To evaluate efficacy of treating a metabolic disease induced by a high fat diet according to a dose of vesicles derived from *Lactobacillus paracasei,* an experiment was performed by the following method.

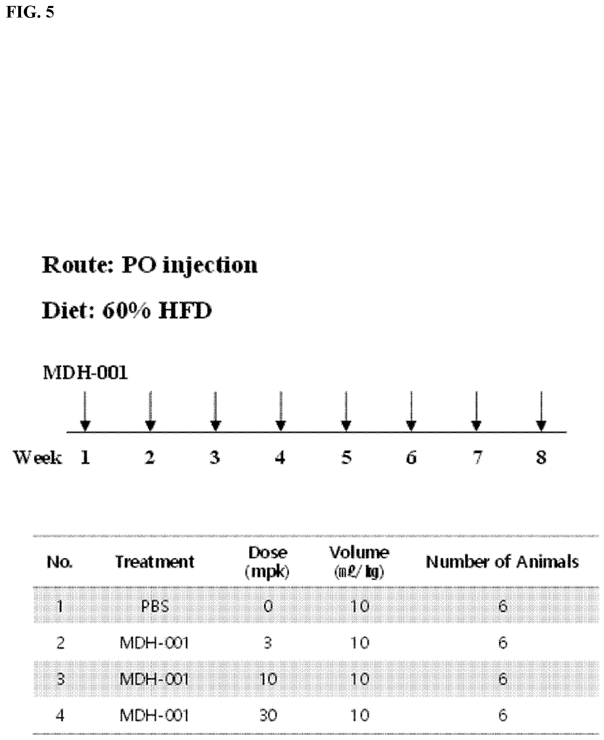

As shown in FIG. 5, a mouse model in which obesity and diabetes was induced by a high fat diet (60% High fat diet; HFD) for 2 weeks were prepared, the vesicles derived from *Lactobacillus paracasei* (MDH-001) obtained in Example 1 were administered at a dose of 3, 10 or 30 mg/kg per weight (g) of a mouse for 8 weeks. Here, as a positive control, a mouse group prepared by administering PBS into the moused model in which obesity and diabetes was induced by a high fat diet (60% High fat diet; HFD) for 2 weeks was used. While administering the vesicles every 2 days, body weights were measured.

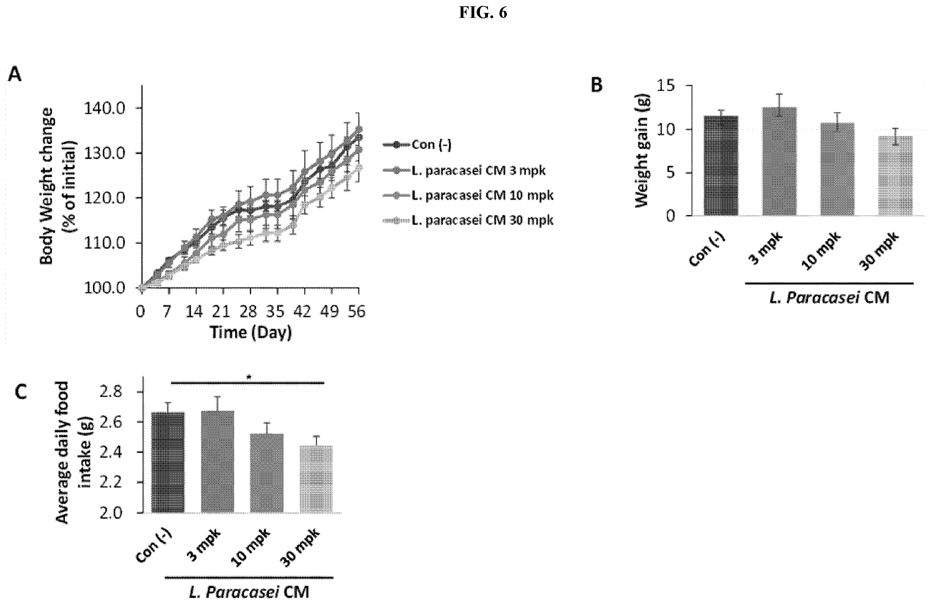

As shown in FIG. 6, it was confirmed that the greater the dose of the vesicles administered for 8 weeks, the clearer the effect of losing body weight, and in the mouse group administered 30 mg/kg of vesicles, compared to the PBS-administered mouse group, it was confirmed that a body weight was reduced by about 5 to 7%. The effect of reducing food intake in a dose-dependent manner of the administered vesicles was also confirmed.

From the above result, it was confirmed that the vesicles derived from *Lactobacillus paracasei* inhibit obesity in a dose-dependent manner, and are associated with a decrease in food intake.

### Example 6. Effect of vesicles derived from Lactobacillus paracasei on glucose metabolism in myocytes

To evaluate the effect of vesicles derived from *Lactobacillus paracasei* on expression of glucose transporter type 4 (GLUT4) in myocytes, myocytes were treated in vitro with 10 µg/ml of vesicles derived from *Lactobacillus paracasei* (L-EVs) for 1 hour. Afterward, a degree of expression of GLUT4 on the cell membrane was assessed by o-phenylenediamine (OPD) assay. As controls, vesicles derived from *E. coli* (EcEVs) and metformin were used.

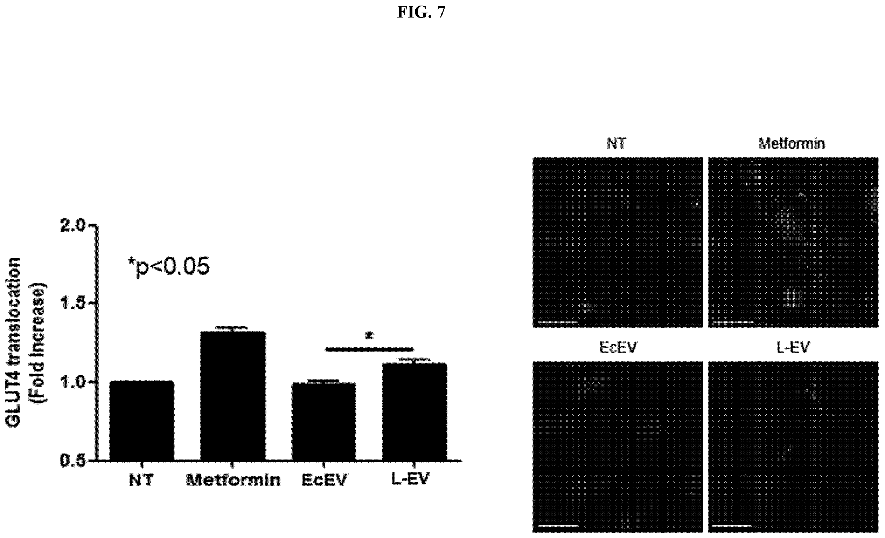

As a result, as shown in FIG. 7, it was confirmed that the GLUT4 expression was not increased by vesicles derived from *E. coli* (EcEVs), but increased by vesicles derived from *Lactobacillus paracasei* and metformin.

In addition, to evaluate an effect of vesicles derived from *Lactobacillus paracasei* on glucose metabolism in myocytes, myocytes were treated in vitro with 10 µg/ml of vesicles derived from *Lactobacillus paracasei* for 1 hour. Afterward, glucose uptake was confirmed with radioactive isotopes.

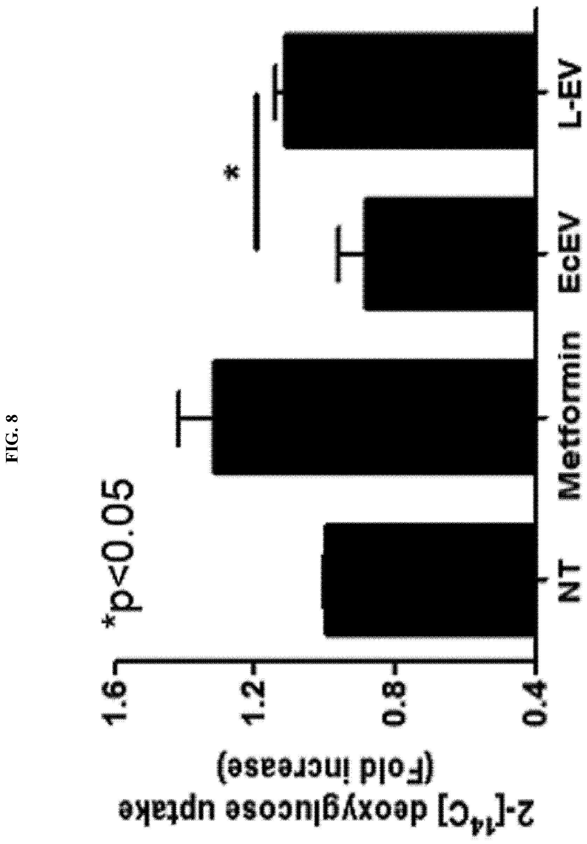

As a result, as shown in FIG. 8, glucose uptake was not increased by vesicles derived from *E*. *coli* (EcEVs), but increased by vesicles derived from *Lactobacillus paracasei* (L-EVs) and metformin.

From the above result, it can be seen that the vesicles derived from *Lactobacillus paracasei* inhibit insulin resistance in an organ on which insulin acts such as myocytes, thereby increasing insulin reactivity.

### Example 7. Effect of vesicles derived from Lactobacillus paracasei on AMPK activation in myocytes

During fasting or exercise, energy in myocytes is depleted, so ATP production decreases and AMP production increases. Here, it is well known that activation of AMPK signaling increases homeostasis to metabolic stress in cells. To evaluate whether the obesity and insulin resistance inhibitory effect by the vesicles derived from *Lactobacillus paracasei* is associated with AMPK signaling, an experiment was performed by the following method. That is, myocytes were treated in vitro with vesicles derived from *Lactobacillus paracasei* (L-EVs) at a concentration of 0, 0.1, 1 or 10 µg/ml for 1 hour. Afterward, a level of AMPK phosphorylation (pAMPK), which is an AMPK signaling indicator, was measured by western blotting. As control drugs, insulin and metformin were used.

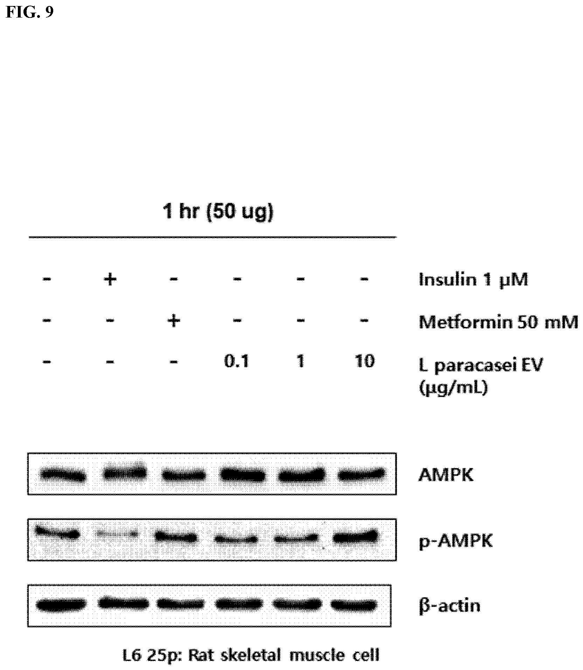

As a result, as shown in FIG. 9, pAMPK expression was inhibited by the control drug, insulin, and increased by metformin. In addition, when vesicles derived from *Lactobacillus paracasei* were treated, pAMPK expression was increased in a vesicle concentration-dependent manner. This means that the obesity and insulin resistance inhibitory effect of the vesicles derived from *Lactobacillus paracasei* is closely related to AMPK signaling that maintains cell homeostasis by metabolic stress.

### Example 8. Effect of vesicles derived from Lactobacillus rhamnosus on AMPK activation in myocytes

To evaluate whether an obesity and insulin resistance inhibitory effect by vesicles derived from *Lactobacillus rhamnosus* is associated with AMPK signaling, an experiment was performed by the following method. That is, myocytes were treated in vitro with vesicles derived from *Lactobacillus rhamnosus* (L-EVs) at 0, 0.1, 1, 10 µg/ml for 1 hour. Afterward, a level of AMPK phosphorylation (pAMPK), which is an AMPK signaling indicator, was measured by western blotting. As control drugs, insulin and metformin were used.

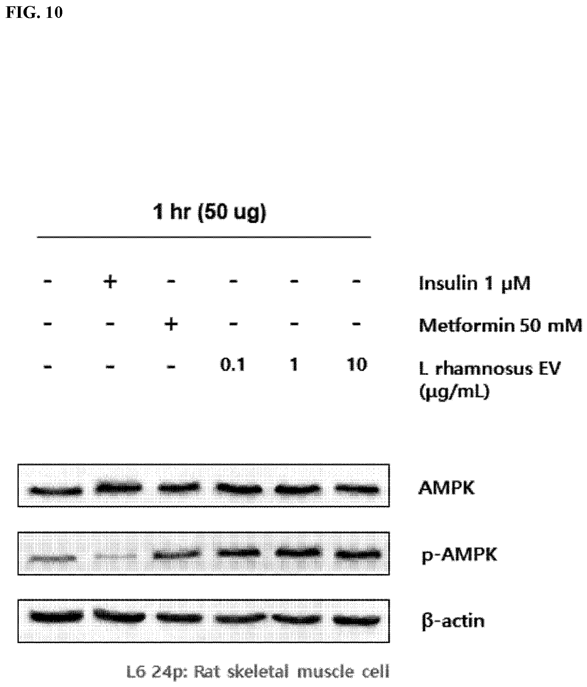

As a result, as shown in FIG. 10, pAMPK expression was inhibited by the control drug, insulin, and increased by metformin. In addition, when vesicles derived from *Lactobacillus rhamnosus* were treated, pAMPK expression was increased in a vesicle concentration-dependent manner. This means that the obesity and insulin resistance inhibitory effect of the vesicles derived from *Lactobacillus rhamnosus* is closely related to AMPK signaling that maintains cell homeostasis by metabolic stress.

### [Industrial Applicability]

The inventors confirmed that, when vesicles derived from bacteria of the genus *Lactobacillus* were administered to a high fat western diet-induced metabolic disease animal model, not only can a body weight and a meal amount be reduced, but also a metabolic disorder by a western diet can be effectively inhibited, and homeostasis to metabolic stress was increased by activating AMPK signaling in myocytes. Therefore, the vesicles derived from bacteria of the genus *Lactobacillus* according to the present invention can be effectively used in development of pharmaceuticals or health functional foods for preventing, improving symptoms or treating metabolic diseases and muscle diseases, and thus have industrial applicability.

Embodiments of the invention are also described in the claims of the International application as filed (PCT/KR2021/007369):
[Embodiment 1]
   A pharmaceutical composition for preventing or treating a metabolic disease or a muscle disease, comprising vesicles derived from bacteria of the genus *Lactobacillus* as an active ingredient.
[Embodiment 2]
   The pharmaceutical composition of embodiment 1, wherein the bacteria of the genus *Lactobacillus* is *Lactobacillus paracasei* or *Lactobacillus rhamnosus.*
[Embodiment 3]
   The pharmaceutical composition of embodiment 1, wherein the metabolic disease is selected from the group consisting of obesity, metabolic syndrome, hyperinsulinemia, hyperlipidemia, hypertriglyceridemia, hypercholesterolemia, dyslipidemia, diabetes, diabetic nephropathy, diabetic retinopathy, fatty liver, non-alcoholic steatohepatitis and arteriosclerosis.
[Embodiment 4]
   The pharmaceutical composition of embodiment 1, wherein the muscle disease is one or more selected from the group consisting of atony, muscular atrophy, muscular dystrophy, myasthenia, cachexia and sarcopenia.
[Embodiment 5]
   The pharmaceutical composition of embodiment 1, wherein the vesicles have an average diameter of 10 to 1000 nm.
[Embodiment 6]
   The pharmaceutical composition of claim 1, wherein the vesicles are isolated from culture solution of bacteria of the genus *Lactobacillus.*
[Embodiment 7]
   The pharmaceutical composition of embodiment 1, wherein the vesicles are isolated from a food prepared by adding bacteria of the genus *Lactobacillus.*
[Embodiment 8]
   The pharmaceutical composition of embodiment 1, wherein the vesicles are naturally or artificially secreted from bacteria of the genus *Lactobacillus.*
[Embodiment 9]
   A food composition for preventing or alleviating a metabolic disease or a muscle disease, comprising vesicles derived from bacteria of the genus *Lactobacillus* as an active ingredient.
[Embodiment 10]
   A food composition for improving a muscle function, comprising vesicles derived from bacteria of the genus *Lactobacillus* as an active ingredient.
[Embodiment 11]
   A food composition for enhancing exercise performance capacity, comprising vesicles derived from bacteria of the genus *Lactobacillus* as an active ingredient.
[Embodiment 12]
   A method for preventing or treating a metabolic disease or a muscle disease, the method comprising administering a composition comprising vesicles derived from bacteria of the genus *Lactobacillus* as an active ingredient to an individual.
[Embodiment 13]
   A use of a composition comprising vesicles derived from bacteria of the genus *Lactobacillus* as an active ingredient for preventing or treating a metabolic disease or a muscle disease.
[Embodiment 14]
   A use of vesicles derived from bacteria of the genus *Lactobacillus* for preparing a drug for preventing or treating a metabolic disease or a muscle disease.
[Embodiment 15]
   A method for improving a muscle function or enhancing exercise performance capacity, the method comprising administering a composition comprising vesicles derived from bacteria of the genus *Lactobacillus* as an active ingredient to an individual.
[Embodiment 16]
   A use of a composition comprising vesicles derived from bacteria of the genus *Lactobacillus* as an active ingredient for improving a muscle function or enhancing exercise performance capacity.
[Embodiment 17]
   A use of vesicles derived from bacteria of the genus *Lactobacillus* for preparing a drug for improving a muscle function or enhancing exercise performance capacity.

The above-described description of the present invention is provided for illustrative purposes, and those of ordinary skill in the art to which the present invention pertains will understand that the present invention can be easily modified into other specific forms without changing the technical spirit or essential features of the present invention, as defined in the following claims. Therefore, it should be understood that the above-described Examples are illustrative only in all aspects and are not restrictive.

## Claims

1. A pharmaceutical composition for use in preventing or treating a metabolic disease or a muscle disease, comprising isolated vesicles derived from *Lactobacillus rhamnosus* as an active ingredient.

2. The pharmaceutical composition for use of claim 1, wherein the metabolic disease is selected from the group consisting of obesity, metabolic syndrome, hyperinsulinemia, hyperlipidemia, hypertriglyceridemia, hypercholesterolemia, dyslipidemia, diabetes, diabetic nephropathy, diabetic retinopathy, fatty liver, non-alcoholic steatohepatitis and arteriosclerosis.

3. The pharmaceutical composition for use of claim 1, wherein the muscle disease is one or more selected from the group consisting of atony, muscular atrophy, muscular dystrophy, myasthenia, cachexia and sarcopenia.

4. The pharmaceutical composition for use of any one of claims 1 to 3, wherein the vesicles have an average diameter of 10 to 1000 nm.

5. The pharmaceutical composition for use of any one of claims 1 to 4, wherein the vesicles are isolated from culture solution of bacteria of *Lactobacillus rhamnosus.*

6. The pharmaceutical composition for use of any one of claims 1 to 4, wherein the vesicles are isolated from a food prepared by adding *Lactobacillus rhamnosus.*

7. The pharmaceutical composition for use of any one of claims 1 to 6, wherein the vesicles are naturally or artificially secreted from *Lactobacillus rhamnosus.*

8. A food composition for use in preventing or alleviating a metabolic disease or a muscle disease, comprising isolated vesicles derived from *Lactobacillus rhamnosus* as an active ingredient.

9. A method for improving a muscle function, comprising administering a composition comprising isolated vesicles derived from *Lactobacillus rhamnosus* as an active ingredient.

10. A method for enhancing exercise performance capacity, comprising administering a composition comprising isolated vesicles derived from *Lactobacillus rhamnosus* as an active ingredient.
